# EUROPÄISCHE PATENTSCHRIFT

(11) **EP 1 207 886 B1**
(45) Veröffentlichungstag und Bekanntmachung des Hinweises auf die Patenterteilung: **16.11.2005**
(21) Anmeldenummer: 00927242.8
(22) Anmeldetag: 24.05.2000
(51) Int. Cl.: A61K 31/5415, A61K 31/538, A61K 31/137, A61K 31/403, A61K 31/496, A61P 31/00, A61P 9/10, A61P 25/28, A61P 31/18

(54) **SPHINGOMYELINASE INHIBITOREN ALS WIRKSTOFFE MIT ANTIAPOPTOTISCHER UND ANTISEPTISCHER WIRKUNG**
SPHINGOMYELINASE INHIBITORS AS AGENTS WITH ANTIAPOPTOTIC AND ANTISEPTIC ACTION
INHIBITEURS DE LA SPHINGOMYELINASE AVEC ACTIVITE ANTIAPOPTOTIQUE ET ANTISEPTIQUE

(30) Priorität: 26.05.1999 DE 19924148
(43) Veröffentlichungstag der Anmeldung: 29.05.2002
(73) Patentinhaber: Biofrontera Pharmaceuticals GmbH, 51377 Leverkusen (DE)
(72) Erfinder: DEIGNER, Hans-Peter, D-68623 Lampertheim (DE); MEISNER, Michael, D-07743 Jena (DE); KINSCHERF, Ralf, D-69198 Schriesheim (DE); BIBAK, Nilofar, D-69118 Heidelberg (DE)
(74) Vertreter: Sternagel, Fleischer, Godemeyer & Partner
(86) Internationale Anmeldenummer: PCT/EP2000/004738
(87) Internationale Veröffentlichungsnummer: WO 2000/072833

(56) Entgegenhaltungen:
- EP-A- 0 354 568
- WO-A-92/09561
- DE-B- 1 051 858
- DE-B- 1 087 605
- WANDEL, CHRISTOPH ET AL: "P-glycoprotein and cytochrome P-450 3A inhibition: dissociation of inhibitory potencies" CANCER RES. (1999), 59(16), 3944-3948 , 1999, XP002155027
- DATABASE CHEMABS [Online] CHEMICAL ABSTRACTS SERVICE, COLUMBUS, OHIO, US; DEIGNER, H. P. ET AL: "Modulating apoptosis: current applications and prospects for future drug development" retrieved from STN Database accession no. 131:96829 XP002155030 & CURR. MED. CHEM. (1999), 6(5), 399-414 ,
- DATABASE CHEMABS [Online] CHEMICAL ABSTRACTS SERVICE, COLUMBUS, OHIO, US; CLAUS, R. ET AL: "Modulation of the ceramide level, a novel therapeutic concept ?" retrieved from STN XP002155031 & CURR. DRUG TARGETS (2000), 1(2), 185-205 ,
- ROMANELLI, M. NOVELLA ET AL: "Synthesis and cardiovascular properties of fluorenyl derivatives related to verapamil" FARMACO (1991), 46(10), 1121-38 , XP002155028
- GUALTIERI, FULVIO ET AL: "Structure-activity relationship studies in the field of calcium(II) antagonists. Effect of modifications at the tetrasubstituted carbon of verapamil-like compounds" J. MED. CHEM. (1985), 28(11), 1621-8 , 1985, XP002155029

## Beschreibung

Die vorliegende Erfindung betrifft pharmazeutische Zusammensetzungen mit antiapoptotischer und antiseptischer Wirkung, die als Hemmstoffe der Sphingomyelinasen wirken. Die erfindungsgemäßen pharmazeutischen Zusammensetzungen sind insbesondere zur Behandlung von Sepsis, Arteriosklerose, neurodegenerativen Erkrankungen, wie z.B. Morbus Alzheimer, und retroviralen Infektionen, wie z.B. HIV-Infektionen, verwendbar.

Die deutsche Auslegeschrift 1 051 858 (veröffentlicht 1959) beschreibt die Herstellung von Abkömmlingen des Phenthiazins sowie die pharmakologische Wirkung dieser Phenthiazinderivate als ganglienblockierend, blutdrucksenkend, nikotinolytisch und sympathicolytisch. Für die Verbindungen werden verschiedene Möglichkeiten der Herstellung beschrieben.

Die deutsche Auslegeschrift 1 087 605 (veröffentlicht 1960) stellt eine Zusatzanmeldung zu der oben genannten DE 1 051 858 dar und behandelt mehr oder weniger denselben Sachverhalt, wobei ganz bestimmte cyangruppenhaltige Phenthiazinderivate betrachtet werden.

Die EP-A-354 568 beschreibt eine andere Art von chemischen Verbindungen, nämlich Catecholderivate, zur Verwendung als Medikament gegen das Fortschreiten von Erkrankungen des Zentralnervensystems oder zur Behandlung von neurologischen Erkrankungen.

Apoptose ist der programmierte Zelltod. Dieser wird z.B. vom Immunsystem genutzt, schädliche Stoffe, wie Viren abzuwehren. Hierzu greifen Virus-spezifische T-Lymphozyten jene Zellen des Körpers an, die Virus-infiziert sind und töten diese ab, indem sie Apoptose-induzierte Proteine freisetzen. Andere Studien ergaben, daß Apoptose auch für die Ausbildung verschiedener Erkrankungen mitverantwortlich ist. Solche Erkrankungen sind z.B. AIDS, Autoimmunerkrankungen und neurodegenerative Erkrankungen.

Der Lipidmediator Ceramid hat einen wesentlichen Anteil bei der Signaltransduktion von oxidativem Stress, der zum Zelltod führen kann. Der Ceramidmediierte Zelltod spielt insofern eine zentrale Rolle bei unterschiedlichsten Erkrankungen wie Sepsis, Arteriosklerose, neurodegenerativen Erkrankungen, wie z.B. Morbus Alzheimer, und retroviralen Infektionen, wie z.B. HIV-Infektionen. Ceramid wird durch die Hydrolyse von Sphingomyelin erzeugt. Der Sphingomyelin-Ceramid-Stoffwechselweg stellt einen Weg dar, Signale von den Rezeptoren auf der Zelloberfläche in den Zellkern unter Verwendung des diffundierenden, lipiden Ceramids als Botenstoff zu übertragen. Die Signalübertragung in die Zellen wird induziert, indem Sphingomyelin durch entsprechende Sphingomyelinspezifische Phospholipasen, den Sphingomyelinasen, hydrolysiert wird.

Ein Ansatz, die intrazelluläre Zunahme der Konzentration an Ceramid zu vermindern, besteht somit in der Hemmung der Sphingomyelinasen. Es gibt jedoch bislang keine klinischen Studien hinsichtlich irgendwelcher Sphingomyelinase-Hemmstoffe.

Somit liegt der vorliegenden Erfindung die Aufgabe zugrunde, Verbindungen und pharmazeutische Zusammensetzungen bereitzustellen, die als Hemmstoffe der Sphingomyelinasen die intrazelluläre Zunahme der Konzentration an Ceramid vermindern sollen, um so die Verwendung solcher pharmazeutischen Zusammensetzungen zur Behandlung von Sepsis, Arteriosklerose, neurodegenerativen Erkrankungen, wie z.B. Morbus Alzheimer, und retroviralen Infektionen, wie z.B. HIV-Infektionen, vorzusehen. Die pharmazeutische Zusammensetzungen sollen hohe Wirkungsselektivität, gleichzeitig aber möglichst geringe Nebenwirkungen bzw. Allgemeintoxizität zeigen.

Diese Aufgabe wird durch die in den Ansprüchen gekennzeichneten Ausführungsformen gelöst.

Insbesondere werden in einer Ausführungsform Verbindungen mit der allgemeinen Formel (I) zur Verwendung als Medikament bereitgestellt 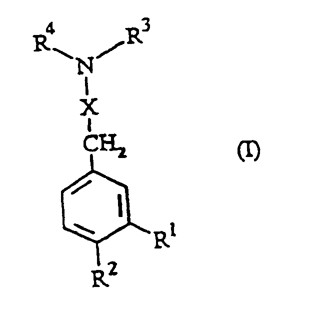 worin die Reste R¹ und R² jeweils unabhängig voneinander ein Wasserstoffatom oder eine Alkoxygruppe mit 1 bis 4 Kohlenstoffatomen bedeuten, X eine Alkylengruppe mit 1 bis 4 Kohlenstoffatomen, die mit einem oder mehreren Fluoratomen substituiert sein kann, oder eine Ketogruppe -CO- bedeutet, die Reste R³ und R⁴ jeweils gleichzeitig für Phenylreste stehen, die unabhängig voneinander mit einem oder mehreren gerad- oder verzweigtkettigen Alkyl- oder Alkoxyresten mit 1 bis 4 Kohlenstoffatomen substituiert sein können, oder der Rest R³ ein Wasserstoffatom oder einen gerad- oder verzweigtkettigen Alkylrest mit 1 bis 4 Kohlenstoffatomen und der Rest R⁴ einen 1-Naphthylalkylenrest -(CH₂)ₙ-C₈H₇, einen [3-(10,11-Dihydro-dibenzo[a,d]cyclohepten-5-yliden)-propyl]-rest -CH₂CH₂CHC₁₅H₁₂, einen N-(1-naphthyl)-N-alkylenaminrest-(CH₂)ₙ-NH-C₈H₇, einen N-(5H-Dibenzo[a,d]cycloheptan-5-yl)-N-alkylenaminrest-(CH₂)ₙ-NH-C₁₅H₁₃, oder einen nachstehenden Rest A bedeuten, 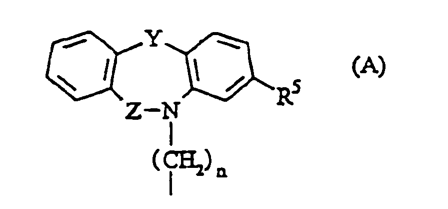 wobei n 1, 2 oder 3 ist, R⁵ ein Wasserstoffatom oder ein Halogenatom bedeutet, Z eine Alkylenbrücke -(CH₂)ₗ-, wobei ℓ 0 oder 1 ist, bedeutet, Y eine Alkylenbrücke -(CH₂)ₘ-_{,} wobei m 0,1 oder 2 ist, ein Sauerstoffatom, oder eine NR⁶-Gruppe bedeutet, wobei der Rest R⁶ ein Wasserstoffatom, einen gerad- oder verzweigtkettigen Alkylrest mit 1 bis 4 Kohlenstoffatomen oder einen unsubstituierten oder substituierten Arylrest bedeutet, oder R³ und R⁴ miteinander unter Einbindung des Stickstoffatoms der Formel (I) einen nachstehenden Piperazinring oder Hexahydropyrimidinring bilden 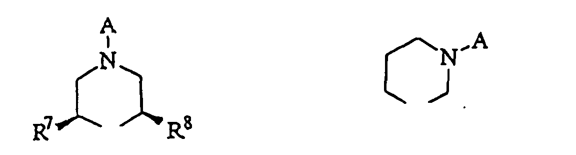 worin der Rest A wie vorgenannt definiert ist und R⁷ und R⁸ jeweils unabhängig voneinander ein Wasserstoffatom oder eine Alkylgruppe mit 1 bis 4 Kohlenstoffatomen bedeuten, oder ein pharmazeutisch verträgliches Salz davon. In einer weiteren Ausführungsform betrifft die Erfindung die Verwendung einer Verbindung mit der allgemeinen Formel (II) 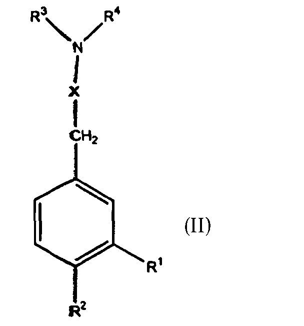 worin die Reste R¹ und R² jeweils unabhängig voneinander ein Wasserstoffatom oder eine Alkoxygruppe mit 1 bis 4 Kohlenstoffatomen bedeuten, X eine Alkylengruppe mit 1 bis 4 Kohlenstoffatomen, die mit einem oder mehreren Fluoratomen substituiert sein kann, oder eine Ketogruppe -CO- bedeutet, die Reste R³ und R⁴ jeweils gleichzeitig für Phenylreste stehen, die unabhängig voneinander mit einem oder mehreren gerad- oder verzweigtkettigen Alkyl- oder Alkoxyresten mit 1 bis 4 Kohlenstoffatomen substituiert sein können, oder der Rest R³ ein Wasserstoffatom oder einen gerad- oder verzweigtkettigen Alkylrest mit 1 bis 4 Kohlenstoffatomen und der Rest R⁴ einen 1-Naphthylalkylenrest -(CH₂)ₙ-C₈H₇, einen [3-(10,11-Dihydro-dibenzo[a,d]cyclohepten-5-yliden)-propyl]-rest-CH₂CH₂CH₁₅H₁₂, einen N-(1-naphthyl)-N-alkylenaminrest-(CH₂)ₙ-NH-C₈H₇, einen N-(5H-Dibenzo[a,d]cycloheptan-5-yl)-N-alkylenaminrest-(CH₂)ₙ-NH-C₁₅H₁₃, oder einen nachstehenden Rest B bedeuten, 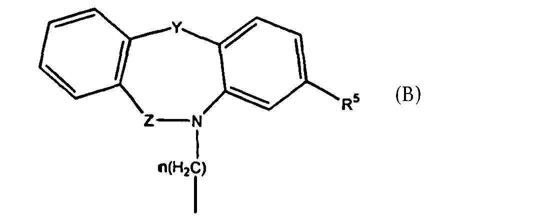 wobei n 1, 2 oder 3 ist, R⁵ ein Wasserstoffatom oder Halogenatom bedeutet, Z eine Alkylenbrücke -(CH₂)ₗ-, wobei l = 0 oder 1 ist, bedeutet. Y eine Alkylenbrücke -(CH₂)ₘ-, wobei m = 0, 1 oder 2 ist, ein Sauerstoffatom, ein Schwefelatom oder eine NR⁶-Gruppe bedeutet, wobei der Rest R⁶ ein Wasserstoffatom, einen gerad- oder verzweigtkettigen Aklylrest mit 1 bis 4 Kohlenstoffatomen oder eine unsubstituierten oder substituierten Arylrest bedeutet, oder R³ und R⁴ miteinander unter Einbindung des Stickstoffatoms der Formel (II) einen nachstehenden Piperazinring oder Hexahydropyrimidinring bilden 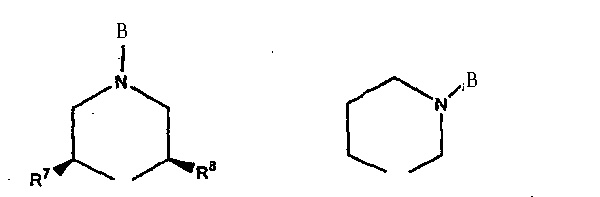 worin der Rest B wie vorgenannt definiert ist und R⁷ und R⁸ jeweils unabhängig voneinander ein Wasserstoffatom oder ein Alkylgruppe mit 1 bis 4 Kohlenstoffatomen bedeuten, oder ein pharmazeutisch verträgliches Salz davon zur Herstellung eines Arzneimittels mit antiapoptotischer und antiseptischer Wirkung. Ein solches Medikament kann eine pharmazeutisch verträglichen Träger und/oder ein pharmazeutisch verträgliches Verdünnungsmittel enthalten.

Die erfindungsgemäßen pharmazeutischen Zusammensetzungen, die mindestens eine Verbindung mit der allgemeinen Formel (I) enthalten, hemmen überraschenderweise in verschiedenen Zelltypen, wie beispielsweise mononukleären Zellen, Fibroblasten und Hepatozyten, die Zunahme der intrazellulären Ceramidkonzentration, die durch Substanzen wie z.B. Tumor-Nekrose-Faktor α, Lipopolysacchariden und oxidierten Lipoproteinen sowie oxidativem Stress, induziert werden kann.

Die in den erfindungsgemäßen pharmazeutischen Zusammensetzungen enthaltenen Verbindungen mit der allgemeinen Formel (I) wirken dabei nachweislich anti-apoptotisch. Zudem zeigten Versuche, daß sie die Mortalität von Mäusen bei induzierter Sepsis reduzieren. Demgemäß stellen die erfindungsgemäßen pharmazeutischen Zusammensetzungen, die mindestens eine Verbindung mit der allgemeinen Formel (I) enthalten, einen neuen Therapieansatz zur Behandlung von beispielsweise Sepsis, Arteriosklerose, neurodegenerativen Erkrankungen, wie z.B. Morbus Alzheimer, und retroviralen Infektionen, wie z.B. HIV-Infektionen, dar.

In einer bevorzugten Ausführungsform der vorliegenden Erfindung ist der Rest B ein (10, 11-Dihydro-dibenzo[b,f]azepin-5-yl)-N-alkylenrest 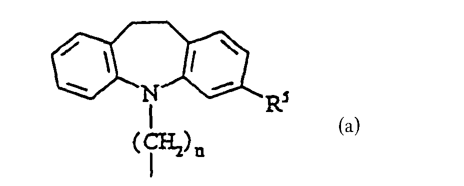 ein Phenothiazin-10-yl-N-alkylenrest 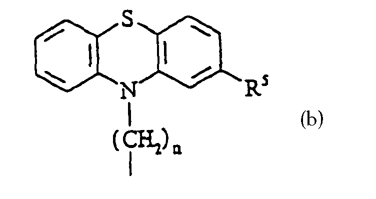 ein (5,6,1 1,12-Tetrahydrodibenzo[b,f]azocin-5-yl)-N-alkylenrest 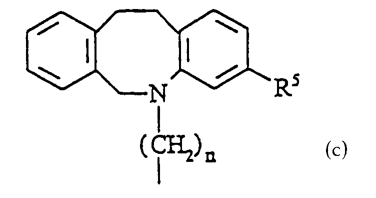 oder ein Carbazol-9-yl-N-alkylenrest

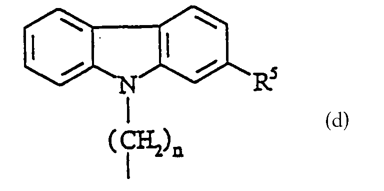

Der Rest (A) ist vorzugsweise einer der Reste (a), (c) oder (d) Der Rest R³ in den Formeln (I) und (II) ist vorzugsweise eine Methylgruppe. Die Reste R¹ und R² in den Formel (I) und (II) sind vorzugsweise jeweils unabhängig voneinander ein Wasserstoffatom oder eine Methoxygruppe. Der Rest R⁵ kann beispielsweise ein Chloratom sein.

In einer Ausführungsform der vorliegenden Erfindung liegt in der pharmazeutischen Zusammensetzung die Verbindung mit der allgemeinen Formel (I) oder (II) vorzugsweise in einer Konzentration von 4 bis 80 Gew.-%, mehr bevorzugt 10 bis 60 Gew.-%, am meisten bevorzugt 15 bis 50 Gew.-%, bezogen auf die Gesamtmenge der pharmazeutischen Zusammensetzung, vor.

Die Konfektionierung der erfindungsgemäßen pharmazeutischen Zusammensetzungen unterliegt keiner besonderen Beschränkung, und sie kann in flüssiger, gelartiger oder fester Darreichungsform vorliegen. Die pharmazeutische Zusammensetzungen können beispielsweise auch in mikroverkapselter Form vorliegen. Die erfindungsgemäßen pharmazeutischen Zubereitungen können neben einer oder mehreren der vorgenannten Verbindungen mit der Formel (I) oder (II) gegebenenfalls einen für den Durchschnittsfachmann bekannten, herkömmlichen pharmazeutisch verträglichen Träger und/oder ein pharmazeutisch verträgliches Verdünnungsmittel enthalten. Die Verabreichung der erfindungsgemäßen pharmazeutischen Zusammensetzungen unterliegt keiner besonderen Beschränkung und kann beispielsweise oral, intravenös, subkutan, intramuskulär, rektal, topisch und/oder äußerlich einem Patienten appliziert werden.

Besonders bevorzugte Verbindungen mit der allgemeinen Formel (I) oder (II), die in den erfindungsgemäßen pharmazeutischen Zusammensetzungen enthalten sein können, sind:
[3-(10,11-Dihydro-dibenzo[b,f]azepin-5-yl)-N-propyl]-[2-(3,4-dimethoxyphenyl)-ethyl]methylamin (V1),
[3-(10,11-Dihydro-dibenzo[b,f]azepin-5-yl)-N-propyl]-[2-(4-methoxyphenyl)-ethyl]methylamin (V2),
[2-(3,4-Dimethoxyphenyl)-ethyl]-[3-(2-chlorphenothiazin-10-yl)-N-propyl]-methylamin (V3), (Formel II)
[2-(4-Methoxyphenyl)-ethyl]-[3-(2-chlorphenothiazin-10-yl)-N-propyl]-methylamin (V4), (Formel II)
[3-(Carbazol-9-yl)-N-propyl]-[2-(3,4-dimethoxyphenyl)-ethyl]methylamin (V5),
[3-(Carbazol-9-yl)-N-propyl]-[2-(4-methoxyphenyl)-ethyl]methylamin (V6),
[2-(3,4-Dimethoxyphenyl)-ethyl]-[2-(phenothiazin-10-yl)-N-ethyl]-methylamin (V7), (Formel II)
[2-(4-Methoxyphenyl)-ethyl]-[2-(phenothiazin-10-yl)-N-ethyl]-methylamin (V8), (Formel II)
[(3,4-Dimethoxyphenyl)-acetyl]-[3-(2-chlorphenothiazin-10-yl)-N-propyl]-methylamin (V9), (Formel II)
N-(1-naphthyl)-N'-[2-(3,4-dimethoxyphenyl)-ethyl]-ethylendiamin (V10),
N-(1-naphthyl)-N'-[2-(4-methoxyphenyl)-ethyl]-ethylendiamin (V11),
N-[2-(3,4-Dimethoxyphenyl)-ethyl]-N-[1-naphthylmethyl]amin (V12),
N-[2-(4-Methoxyphenyl)-ethyl]-N-[1-naphthylmethyl]amin (V13),
[3-(10,11-Dihydro-dibenzo[b,f]azepin-5-yl)-N-propyl]-[(4-methoxyphenyl)-acetyl]methylamin (V14),
[2-(10,11-Dihydro-dibenzo[b,f]azepin-5-yl)-N-ethyl]-[2-(3,4-dimethoxyphenyl)-ethyl]methylamin (V15),
[2-(10,11-Dihydro-dibenzo[b,f]azepin-5-yl)-N-ethyl]-[2-(4-methoxyphenyl)-ethyl]methylamin (V16),
[2-(10,11-Dihydro-dibenzo[b,f]azepin-5-yl)-N-ethyl]-[(4-methoxyphenyl)-acetyl]methylamin (V17),
N-[2-(Carbazol-9-yl)-N-ethyl]-N'-[2-(4-methoxyphenyl)-ethyl]piperazin (V18),
1-[3-(Carbazol-9-yl)-N-propyl]-4-[2-(4-methoxyphenyl)-ethyl]-3,5-dimethyl-piperazin (V19);
[2-(4-Methoxyphenyl)-ethyl]-[3-(phenoxazin-10-yl)-N-propyl]-methylamin(V20),
[3-(5,6,11, 12-Tetrahydrodibenzo[b,f]azocin)-N-propyl]-[3-(4-methoxyphenyl)-propyl]methylamin (V21),
N-(5H-Dibenzo[a,d]cycloheptan-5-yl)-N'-[2-(4-methoxyphenyl)-ethyl]-propylendiamin (V22),
[2-(Carbazol-9-yl)-N-ethyl]-[2-(4-methoxyphenyl)-ethyl]methylamin (V23),
[3-(Carbazol-9-yl)-N-propyl]-(4-methoxybenzyl)-methylamin (V30)
[3-(10,11-Dihydro-dibenzo[a,d]cyclohepten-5-yliden)-propyl]-[2-(4-methoxyphenyl)-ethyl]methylamin (V31),
(4-Carbazol-9-yl-1,1,2,2,3,3,4,4-octafluorbutyl)-[2-(4-methoxyphenyl)-ethyl]-methylamin (32),
N-[2-(4-Methoxyphenyl)-ethyl]-N-methyl-N',N'-diphenylpropan-1,3-diamin (V34),
N-[2-(3,4-Dimethoxyphenyl)-ethyl]-N-methyl-N',N'-diphenylpropan-1,3-diamin (V35),
10-(3-{4-[2-(4-Methoxyphenyl)-ethyl]-3,5-dimethylpiperazin-1-yl}-propyl)-10H-phenoxazin (V36),
[2-(3,4-Dimethoxyphenyl)-ethyl]-methyl-(phenoxazin-10-yl-propyl)amin (V37),
[3-(10,11-Dihydro-dibenzo[a,d]cyclohepten-5-yliden)-propyl]-[2-(3,4-dimethoxyphenyl)-ethyl]methylamin (V38),
N-(10,11-Dihydro-5H-dibenzo[a,d]cyclohepten-5-yl)-N'-[2-(3,4-dimethoxyphenyl)-ethyl]-hexahydropyrimidin (V41) und
10-(3-{4-[2-(3,-Dimethoxyphenyl)-ethyl]-3,5-dimethylpiperazin-1-yl}-propyl)-9H-carbazol (V42).

Insbesondere für den Fall, daß der Rest A ein (10,11-Dihydro-dibenzo[b,f]azepin-5-yl)-N-alkylenrest, ein Phenothiazin-10-yl-N-alkylenrest, ein (5,6,11,12-Tetrahydrodibenzo[b,f]azocin-5-yl)-N-alkylenrest oder ein Carbazol-9-yl-N-alkylenrest ist, lassen sich die in der erfindungsgemäßen pharmazeutischen Zusammensetzung enthaltenen Verbindungen mit der Formel (I) oder (II) beispielsweise nach dem folgenden Umsetzungsschema, ohne darauf beschränkt zu sein, bereitstellen, wobei n, X, Y, Z, R¹, R² und R⁵ wie vorgenannt definiert sind.

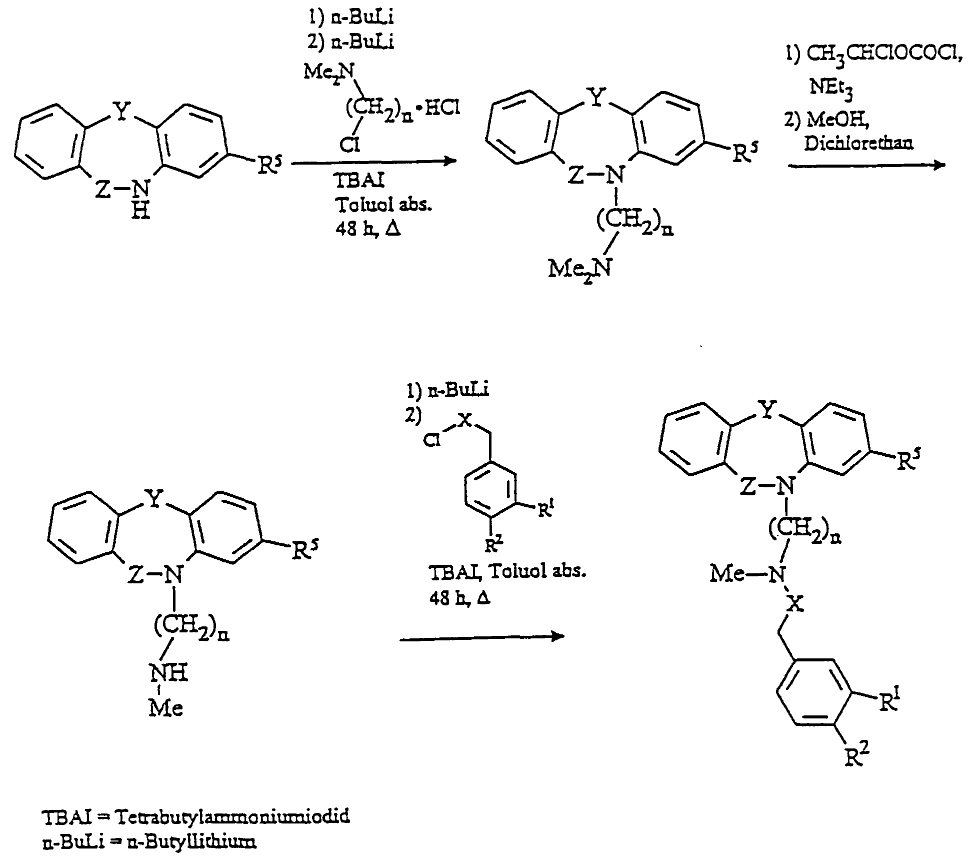

Die vorliegende Erfindung wird nachstehend durch Beispiele detaillierter erläutert:

### LDL-Herstellung

LDL wurde von gesunden, freiwilligen Testpersonen durch sequentielle Flotation in einer Ultrazentrifuge bei vorbestimmten Dichten erhalten, wie in Havel et al. (J. Clin. Invest. 34, 3145 (1955)) bzw. Deigner et al. (Arzneim.-Forsch./Drug Res. 44, 956, (1994)) beschrieben. Für den Apoptose-Test wurde LDL durch Dialyse gegen Tris-HCl-Puffer, der 20 µMol FeCl₃ enthielt, für 48 Stunden minimal modifiziert (mmLDL), wie von Watson et al. (J. Clin. Invest. 95, 774 (1995) beschrieben.

### Zellkultur und Apoptose-Test

Menschliche, mononucleäre, periphere Blutzellen aus venösem Blut wurden isoliert, gezüchtet und, wie in Kinscherf et al. (The FASEB J. 11, 1317 (1997); The FASEB J. 12, 461 (1998)) beschrieben, bis zum Differenzieren zu Mφ belassen. Differenzierte Mφ wurden mit Lipoproteinmangelserum (P>1,21, durch Ultrazentrifugieren von fötalem Kälberserum hergestellt, wie in Havel et al. beschrieben) für 24 Stunden vor der Testdurchführung gezüchtet, mit der entsprechenden Verbindung für 30 Minuten bei den angeführten Konzentrationen vorbehandelt und anschließlich mLDL oder rekombinantem TNF α (3 ng/ml) in dem gleichen Medium ausgesetzt. Die Bestimmung der Apoptose wurde durch Zählen von YOPRO-1-gefärbten apoptotischen Zellen, wie in Idziorek et al. (J. Immunol. Methods 185, 249 (1995)) beschrieben, nach vier Stunden durchgeführt.

### SMase-Inhibierung

Menschliche Makrophagen wurden mit entsprechenden, in den erfindungsgemäßen Zusammensetzungen enthaltenen Verbindungen für 30 Minuten und anschließend zwei Stunden mit oxidiertem LDL (27 µg/ml) vorbehandelt. Anschließend wurde das Medium entfärbt, die Zellen mit vorgekühltem (4°C) PBS gewaschen und 15 Minuten mit Lysepuffer (NaAc, 250 mMol, pH 5,0, TritonX-100 0,2%) bei 4°C behandelt und zentrifugiert (18.000 g). Die Überstände (5 µl) wurden mit ARB-Puffer (NaAc, 250 mMol, pH 5,0) unter Erhalten einer Proteinkonzentration von 80 µg/ml verdünnt, 45 Minuten mit BODIPY®-FL-C₅-sphingomyelin (8 nM) bei 37°C inkubiert. Anschließend wurden 150 µl Methanol (interner Standard: C₅-BODIPY®-Säure) zugegeben. Die Proben wurden einer HPLC-Analyse (50% MeOH/10% Tris-HCl, pH 7,5, 10 mMol, 2 ml/min, E. Merck Lichrospher 125-4, 100-5 µMol RP-18, Fluoreszenzdetektion 503_{Ex}/512_{Em}) unterworfen.

Die Ceramidkonzentrationen in den Zellen wurden nach Diacylglycerolkatalysierter Phosphorylierung, wie in Kinscherf et al. (FEBS, 405, 55 (1997)) und Dressler et al. (J. Biol. Chem. 265, 14917 (1990)) beschrieben, bestimmt.

Die nachstehende Tabelle zeigt die Apoptose-inhibierende Wirkung hinsichtlich der erforderlichen Konzentration zur Hemmung der Apoptose um 50% (IC-50) sowie die Hemmung der Sphingomyelinaseaktivität in den Zellen als erforderliche Konzentration zur Hemmung um 50% (IC-50) von einigen beispielhaft ausgewählten Verbindungen mit der vorgenannten allgemeinen Formel (I), die in den erfindungsgemäßen pharmazeutischen Zusammensetzungen enthalten sein können:

| Verbindung | Apoptose (IC-50, µM) | Hemmung der sauren Sphingomyelinase (Zellen) (IC-50, µM) |
|---|---|---|
| V2 | 3 | 15 |
| V3 | 2 | 6 |
| V4 | 5 | 101 |
| V5 | 6 | 8 |
| V6 | 0,5 | 9 |
| V8 | 3 | 9 |
| V15 | 1 | 14 |
| V16 | 7 | 16 |
| V18 | 0,5 | 33 |
| V19 | 2, 5 | 9 |
| V20 | 5 | 6 |
| V21 | 11 | 17 |
| V22 | 3 | 10 |
| V23 | 4 | 8 |
| V30 | 1,5 | 30 |
| V31 | 1,5 | 20 |
| V32 | 18 | |
| V34 | 3 | |
| V35 | 6 | 63 |
| V36 | 1 | |
| V37 | 2,5 | |
| V38 | 6 | |
| V41 | 7 | |
| V42 | 4 | |

Im Rahmen der vorliegenden Erfindung wurde festgestellt, daß auch 3-(4-Methoxybenzyl)-1H-chinazolin-2,4-dion (Apoptose (IC-50, µM) = 3), 1,3-Bis-(4-methoxybenzyl)-1H-chinazolin-2,4-dion (Apoptose (IC-50, µM) = 0,5) und 9-(4-Methoxybenzyl-9H-carbazol) (Apoptose (IC-50, µM) = 1) als Hemmstoffe der Sphingomyelinasen wirken.

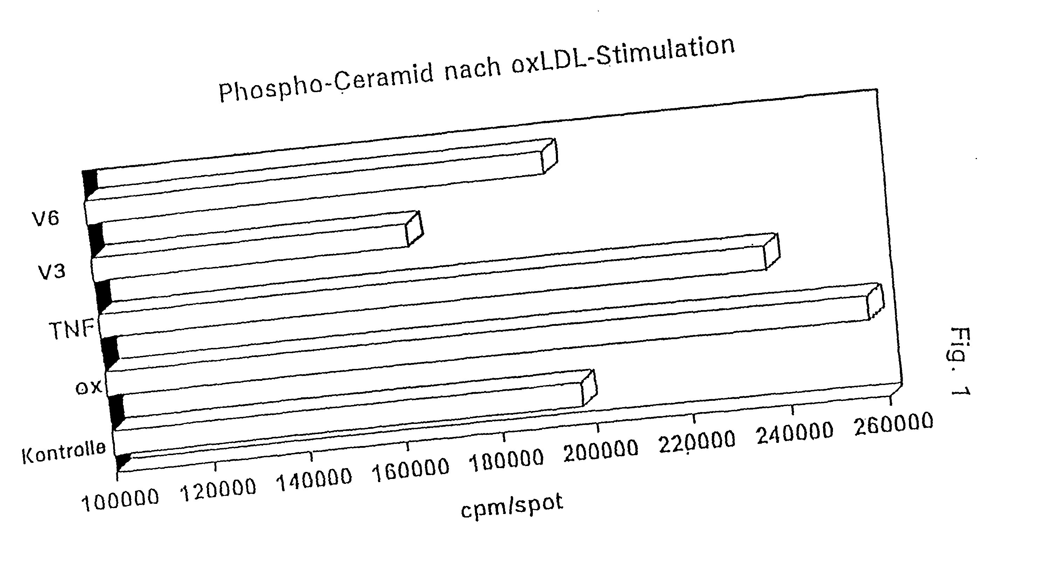

Figur 1 zeigt in einem Diagramm die Phospho-Ceramid-Konzentration (cpm/spot) nach ox. LDL-Stimulierung zum einen ohne (ox) und zum anderen unter Zugabe entsprechender Verbindungen mit der allgemeinen Formel (I), die in den erfindungsgemäßen pharmazeutischen Zusammensetzungen enthalten sein können, nämlich [2-(3,4-Dimethoxyphenyl)-ethyl]-[3-(2-chlorphenothiazin-10-yl)-N-propyl]-methylamin (V3) und [3-(Carbazol-9-yl)-N-propyl]-[2-(4-methoxyphenyl)-ethyl]methylamin (V6). Ferner ist auch die Phospho-Ceramid-Konzentration in Folge der Stimulation durch TNF angegeben.

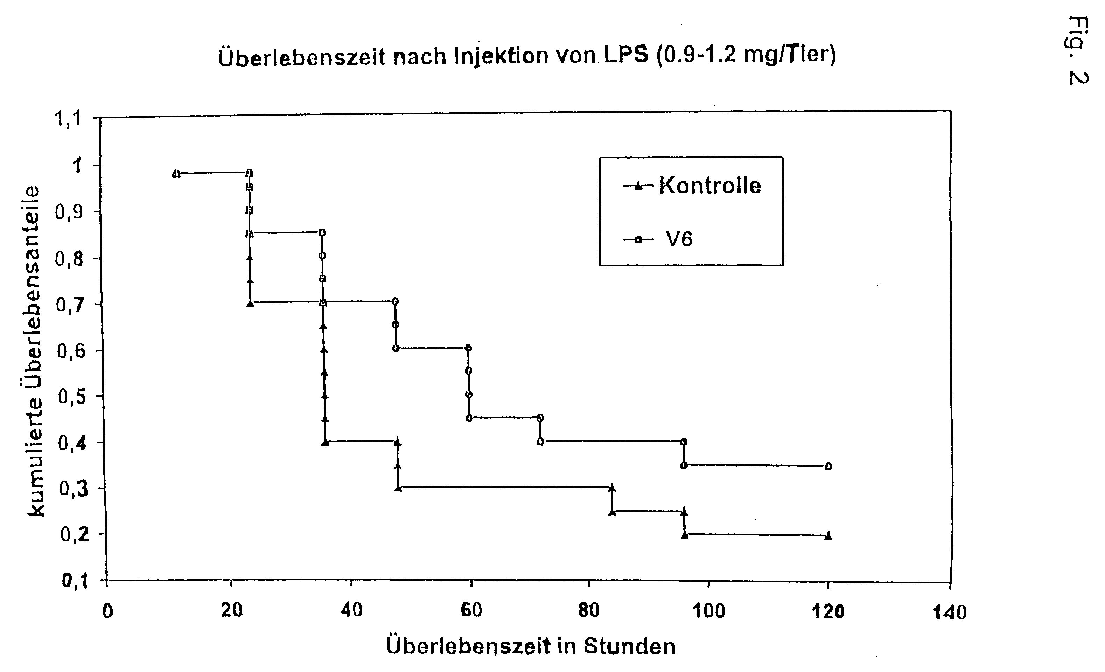

Figur 2 zeigt in einem Diagramm die Überlebenszeit weiblicher NMRI-Mäuse (n=48) nach Injektion von Lipopolysaccharid (LPS, 0,9-1,2 mg, *i*.*p*), wobei die Hälfte der Tiere (n =14) 10 Minuten mit einer erfindungsgemäßen pharmazeutischen Zusammensetzung, die 20 mg/kg [3-(Carbazol-9-yl)-N-propyl]-[2-(4-methoxyphenyl)-ethyl]methylamin (V6) enthielt, vorbehandelt (*i*.*p*.) wurden.

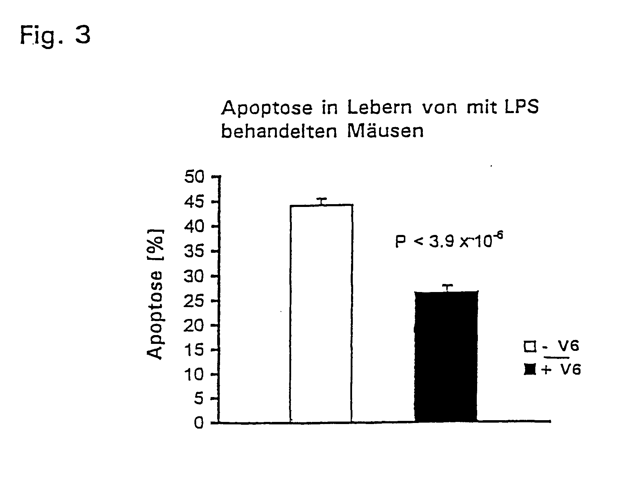

Figur 3 zeigt den prozentualen Anteil apoptotischer Zellen, nachdem die Lebern einiger Versuchstiere - ein Teil war unvorbehandelt, ein anderer Teil war mit der vorgenannten erfindungsgemäßen pharmazeutischen Zusammensetzung, die 20 mg/kg [3-(Carbazol-9-yl)-N-propyl]-[2-(4-methoxyphenyl)-ethyl]methylamin (V6) enthielt (jeweils n = 6), vorbehandelt - entnommen wurden, in Isopentan (N₂-gekühlt) schockgefroren und auf einem Kryostaten geschnitten wurden. Anschließend wurden apoptotische Zellen mit der TUNEL-Methode identifiziert. Der prozentuale Anteil apoptotischer Zellen wurde mittels Computerunterstützter Morphometrie quantifiziert.

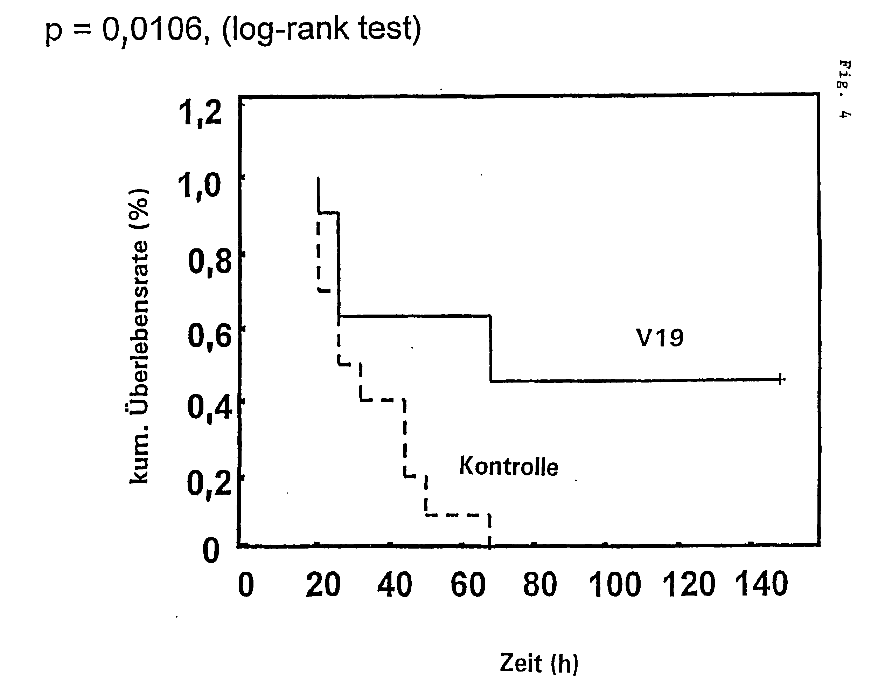

Figur 4 zeigt in einem Diagramm die Überlebensrate weiblicher NMRI-Mäuse (Alter 15 Wochen) nach Injektion von Lipopolysaccharid, wobei nahezu die Hälfte der Tiere (n = 8) (Kontrolle n = 7) mit einer erfindungsgemäßen pharmazeutischen Zusammensetzung, die 1-[3-(Carbazol-9-yl)-N-propyl]-4-[2-(4-methoxyphenyl)-ethyl]-3,5-dimethylpiperazin (V19) enthielt, vorbehandelt wurden (Verabreichung 15 min vor der Verabeichung von LPS: 1 ml der Verbindung V19, gelöst in Thomaejonin *i.p.*, 1 ml Thomaejonin in der Kontrolle; anschließend (t=0) 0,9 mg LPS, weitere 1 ml der Verbindung V19 nach 1 h).

### Mechanismus von anti-apoptotischen Substanzen: aSmase-Inhibitoren

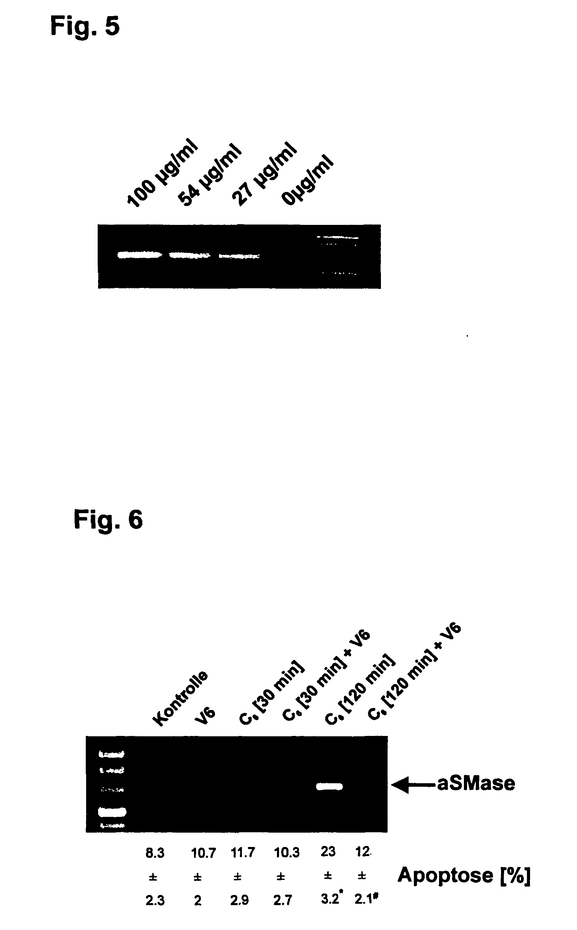

Fig. 5 zeigt die RT-PCR von aSMase in Mφ. Es zeigt sich eine Expression von aSMase (687 bp) nach Inkubation (4 h) von Mφ mit steigenden mmLDL-Konzentrationen.

Fig. 6 zeigt die Wirkung einer Vorbehandlung von Mφ mit einer Zusammensetzung die [3-(Carbazol-9-yl)-N-propyl]-[2-(4-methoxyphenyl)-ethyl]-methylamin (V6) enthält (2 µM, 30 min), auf die aSMase-Expression und die Apoptose in Mφ (Prozentanteil von apoptotischen Mφ, bestimmt durch YOPRO-1-Färbung nach Inkubation mit Medium oder C₆-Ceramid (10 µM) mit oder ohne Vorbehandlung mit der Verbindung V6; Daten sind als Mittelwert ± SEM von drei Experimenten angegeben. *, P ≤ 0,05 vs. Kontrolle oder #, P ≤ 0,05 vs. mit C₆-Ceramid behandelten Zellen (120 min)).

Eine PCR-Analyse ergab, daß mmLDL den Anteil von aSmase-mRNA in Mφ deutlich erhöht (vgl. Fig. 5). Sequenzieren des 687 bp umfassenden Produkts bestätigte die Amplifizierung des entsprechenden aSMase-Fragments. Parallel mit einer Stimulierung der aSmase-Transkription wurde ein signifikanter Apoptose-Anstieg festgestellt (0 µg/ml mmLDL, 7 ± 2,1 % [apoptotische Zellen]; 27 µg/ml, 20,7± 2,7 %; 54 µg/ml, 29,3±3,8 %; 100 µg/ml, 37±4 %. Daten sind als Mittelwert ± SEM von drei Experimenten angegeben).

Es gibt insofern einen deutlichen Beleg dafür, daß die mmLDL-vermittelte Apoptose die Erzeugung von Ceramid einschließt. Daher wurde untersucht, ob Ceramid selbst die Bildung des Ceramid liefernden Enzyms, aSMase, stimuliert. Aus Fig. 5 ist zum ersten Male ersichtlich, daß mmLDL, aber auch Ceramid allein die aSMase-Expression induzieren (vgl. Fig. 6), wie es auch für H₂O₂ (30 µM, nicht gezeigt) der Fall ist. Es wurde festgestellt, daß beispielsweise die Vorbehandlung von Mφ mit einer Zusammensetzung, welche [3-(Carbazol-9-yl)-N-propyl]-[2-(4-methoxyphenyl)-ethyl]methylamin (V6) enthält, sowohl die Ceramid-vermittelte aSMase-Transkription und die Induktion von Apoptose (vgl. Fig. 6) als auch die Aktivierung des Enzyms verhinderte, wie in Zelllysaten gemessen. Ähnliche Ergebnisse wurden in mit mmLDL behandelten Mφ erhalten (Daten nicht gezeigt). Ferner wurde festgestellt, daß die Vorbehandlung von Mφ mit einer Zusammensetzung, die [3-(Carbazol-9-yl)-N-propyl]-[2-(4-methoxyphenyl)-ethyl]-methylamin (V6) enthält, ebenso einen durch mmLDL (27 µg/ml) initiierten Anstieg der aSMase-Aktivität in Zelllysaten vollständig verhindert (Basis: Aktivität 2,18 nmol/h x mg)]).

[3-(Carbazol-9-yl)-N-propyl]-[2-(4-methoxyphenyl)-ethyl]-methylamin (V6), eine Verbindung ohne Redoxaktivität, sowie andere erfindungsgemäße Zusammensetzungen, die eine der vorgenannten Verbindungen wie z.B. [2-(3,4-Dimethoxyphenyl)-ethyl]-[3-(2-chlorphenothiazin-10-yl)-N-propyl]-methylamin (V3) enthalten, inhibieren die mmLDL- oder Ceramid-induzierte Enzyminduktion, die Stimulierung der Ceramidbildung und vermindert parallel dazu den programmierten Zelltod in Mφ. Bei etwas erhöhten Konzentrationen kann eine vollständige Inhibierung der aSMase-Expression erzielt werden. Ohne daran gebunden zu sein, kann somit angenommen werden, daß, wenn direkte Inhibierungswirkungen hinsichtlich des isolierten Enzyms nicht vorliegen, die erfindungsgemäßen Zusammensetzungen durch Inhibierung der aSMase-Transkription wirken.

### Methoden

Menschliche, mononucleäre, periphere Blutzellen aus venösem Blut wurden mittels Ficoll-Paque (Sigma, Deisenhofen, BR Deutschland) Dichtegradientenzentrifugierung isoliert.
Mφ wurden mmLDL (27 µg/ml), C₆-Ceramid (10 µM) bzw. H₂O₂ (30 µM) unterworfen. Apoptotische Zellen wurden mittels YOPRO-1-Färbung (s. Idziorek et al, J. Immunol. Methods, 185, 1995, 249-258) und/oder mittels TUNEL-Technik (Quantum Appligene, Heidelberg, BR Deutschland) identifiziert. Die Bestimmung der Apoptose (Prozentanteil apoptotischer Zellen) wurde durch Zählen von YOPRO-1-gefärbten apoptotischen Zellen unter Verwendung eines Mikroskops, ausgestattet mit einer Quecksilberlampe und einer Fluoreszenzeinheit in Kombination mit einem Computer-unterstützten Morphometriesystem, quantifiziert.

### RT-PCR

Für die Amplifizierung der aSMase waren die Primer und die Bedingungen wie folgt:
5'-CAGGGTTCCTGGCTGGGCAGCA-3' (vorwärts) und 5'-GGTCCTGGACCATGAGACCTAC-3', 94°C, 60°C, 72°C, jeweils 1 min, 40 Zyklen (Sequenzanalyse des gereinigten PCR-Produkts durch das Deutsche Krebsforschungszentrum, Heidelberg, BR Deutschland, bestätigte die Amplifizierung des erwarteten aSMase-Fragments).

## Patentansprüche

1. Verbindung mit der allgemeinen Formel (I) 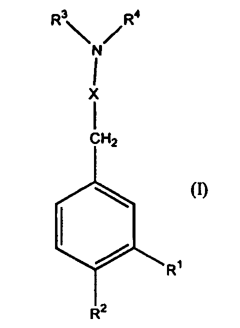 worin die Reste R¹ und R² jeweils unabhängig voneinander ein Wasserstoffatom oder eine Alkoxygruppe mit 1 bis 4 Kohlenstoffatomen bedeuten, X eine Alkylengruppe mit 1 bis 4 Kohlenstoffatomen, die mit einem oder mehreren Fluoratomen substituiert sein kann, oder eine Ketogruppe -CO- bedeutet, die Reste R³ und R⁴ jeweils gleichzeitig für Phenylreste stehen, die unabhängig voneinander mit einem oder mehreren gerad- oder verzweigtkettigen Alkyl- oder Alkoxyresten mit 1 bis 4 Kohlenstoffatomen substituiert sein können, oder der Rest R³ ein Wasserstoffatom oder einen gerad- oder verzweigtkettigen Alkylrest mit 1 bis 4 Kohlenstoffatomen und der Rest R⁴ einen 1-Naphthylalkylenrest -(CH₂)ₙ-C₈H₇, einen [3-(10,11-Dihydro-dibenzo[a,d]cyclohepten-5-yliden)-propyl]-rest-CH₂CH₂CHC₁₅H₁₂, ei nen N-(1-naphthyl)-N-alkylenaminrest-(CH₂)ₙ-NH-C₈H₇, einen N-(5H-Dibenzo[a,d]cycloheptan-5-yl)-N-alkylenaminrest-(CH₂)ₙ-NH-C₁₅H₁₃, oder einen nachstehenden Rest A bedeuten, 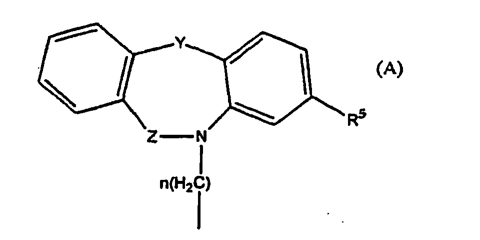 wobei n 1, 2 oder 3 ist, R⁵ ein Wasserstoffatom oder Halogenatom bedeutet. Z eine Alkylenbrücke -(CH₂)ₗ-, wobei l = 0 oder 1 ist, bedeutet, Y eine Alkylenbrücke -(CH₂)ₘ-, wobei m = 0, 1 oder 2 ist, ein Sauerstoffatom oder eine NR⁶-Gruppe bedeutet, wobei der Rest R⁶ ein Wasserstoffatom, einen gerad- oder verzweigtkettigen Alkylrest mit 1 bis 4 Kohlenstoffatomen oder einen unsubstituierten oder substituierten Arylrest bedeutet, oder R³ und R⁴ miteinander unter Einbindung des Stickstoffatoms der Formel (I) einen nachstehenden Piperazinring oder Hexahydropyrimidinring bilden 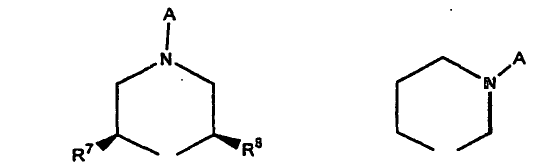 worin der Rest A wie vorgenannt definiert ist und R⁷ und R⁸ jeweils unabhängig voneinander ein Wasserstoffatom oder eine Alkylgruppe mit 1 bis 4 Kohlenstoffatomen bedeuten, oder ein pharmazeutisch verträgliches Salz davon zur Verwendung als Medikament.

2. Verbindung nach Anspruch 1, wobei der Rest A ein (10,11-Dihydrodibenzo[b,f]azepin-5-yl)-N-alkylenrest, ein (5,6,11,12-Tetrahydrodibenzo[b,f]azocin-5-yl)-N-alkylenrest oder ein Carbazol-9-yl-N-alkylenrest ist.

3. Verbindung nach Anspruch 1 oder 2, wobei der Rest R³ eine Methylgruppe ist.

4. Verbindung nach einem der Ansprüche 1 bis 3, wobei die Reste R¹ und R² jeweils unabhängig voneinander ein Wasserstoffatom oder eine Methoxygruppe sind.

5. Verbindung nach einem der Ansprüche 1 bis 4, wobei der Rest R⁵ ein Chloratom ist.

6. Pharmazeutische Zusammensetzung, enthaltend eine Verbindung nach einem der Ansprüche 1 bis 5 und gegebenenfalls einen pharmazeutisch verträglichen Träger und/oder ein pharmazeutisch verträgliches Verdünnungsmittel.

7. Pharmazeutische Zusammensetzung nach Anspruch 6, wobei die Verbindung mit der allgemeinen Formel (I) in einer Konzentration von 4 bis 80 Gew.-%, bezogen auf die Gesamtmenge der pharmazeutischen Zusammensetzung, vorliegt.

8. Pharmazeutische Zusammensetzung nach einem der Ansprüche 6 oder 7, welche in flüssiger, gelartiger oder fester Darreichungsform vorliegt.

9. Verwendung einer Verbindung mit der allgemeinen Formel (II) 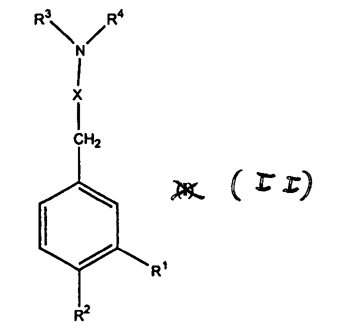 worin die Reste R¹ und R² jeweils unabhängig voneinander ein Wasserstoffatom oder eine Alkoxygruppe mit 1 bis 4 Kohlenstoffatomen bedeuten, X eine Alkylengruppe mit 1 bis 4 Kohlenstoffatomen, die mit einem oder mehreren Fluoratomen substituiert sein kann, oder eine Ketogruppe -CO- bedeutet, die Reste R³ und R⁴ jeweils gleichzeitig für Phenylreste stehen, die unabhängig voneinander mit einem oder mehreren gerad- oder verzweigtkettigen Alkyl- oder Alkoxyresten mit 1 bis 4 Kohlenstoffatomen substituiert sein können, oder der Rest R³ ein Wasserstoffatom oder einen gerad- oder verzweigtkettigen Alkylrest mit 1 bis 4 Kohlenstoffatomen und der Rest R⁴ einen 1-Naphthylalkylenrest -(CH₂)ₙ-C₈H₇, einen [3-(10,11-Dihydro-dibenzo[a,d]cyclohepten-5-yliden)-propyl]-rest-CH₂CH₂CHC₁₅H₁₂, einen N-(1-naphthyl)-N-alkylenaminrest-(CH₂)ₙ-NH-C₈H₇, einen N-(5H-Dibenzo[a,d]cycloheptan-5-yl)-N-alkylenaminrest-(CH₂)ₙ-NH-C₁₅H₁₃, oder einen nachstehenden Rest B bedeuten, 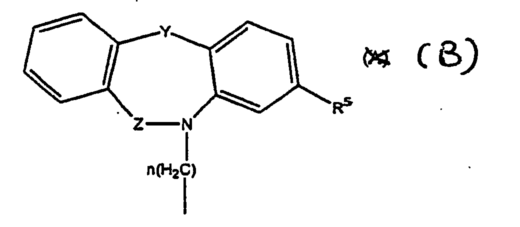 wobei n 1, 2 oder 3 ist, R⁵ ein Wasserstoffatom oder Halogenatom bedeutet. Z eine Alkylenbrücke -(CH₂)ₗ-, wobei l = 0 oder 1 ist, bedeutet, Y eine Alkylenbrücke -(CH₂)ₘ-, wobei m = 0, 1 oder 2 ist, ein Sauerstoffatom, ein Schwefelatom oder eine NR⁶-Gruppe bedeutet, wobei der Rest R⁶ ein Wasserstoffatom, einen gerad- oder verzweigtkettigen Alkylrest mit 1 bis 4 Kohlenstoffatomen oder einen unsubstituierten oder substituierten Arylrest bedeutet, oder R³ und R⁴ miteinander unter Einbindung des Stickstoffatoms der Formel (II) einen nachstehenden Piperazinring oder Hexahydropyrimidinring bilden 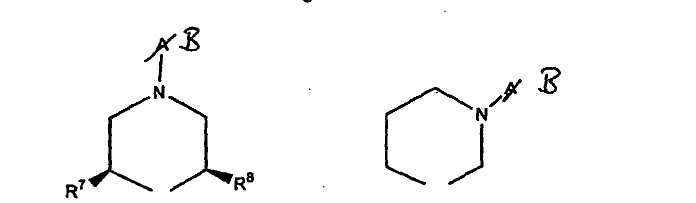 worin der Rest B wie vorgenannt definiert ist und R⁷ und R⁸ jeweils unabhängig voneinander ein Wasserstoffatom oder eine Alkylgruppe mit 1 bis 4 Kohlenstoffatomen bedeuten, oder ein pharmazeutisch verträgliches Salz davon zur Herstellung eines Arzneimittels mit antiapoptotischer und antiseptischer Wirkung.

10. Verwendung gemäß Anspruch 9 zur Herstellung eines Arzneimittels zur Behandlung von neurodegenerativen Erkrankungen, Sepsis, Arteriosklerose und retroviralen Infektionen.

11. Verwendung einer Verbindung gemäß Anspruch 9 oder 10, wobei der Rest B ein (10,11-Dihydro-dibenzo[b.f]azepin-5-yl)-N-alkylenrest, ein (5,6,11,12-Tetrayhdrodibenzo[b,f]azocin-5-yl)-N-alkylenrest, ein Phenothiazin-10-yl-N-alkylenrest oder ein Carbazol-9-yl-N-alkylenrest ist.

12. Verwendung einer Verbindung gemäß Ansprüchen 9 bis 11, wobei der Rest R³ eine Methylgruppe ist.

13. Verwendung einer Verbindung nach einem der Ansprüche 9 bis 12, wobei die Reste R¹ und R² jeweils unabhängig voneinander ein Wasserstoffatom oder eine Methoxygruppe sind.

14. Verwendung einer Verbindung nach einem der Ansprüche 9 bis 13, wobei der Rest R⁵ ein Chloratom ist.

## Claims

1. A compound having the general formula (I): 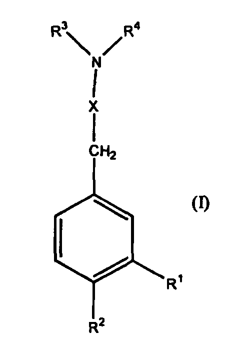 wherein each of the radicals R¹ and R² independently is a hydrogen atom or an alkoxy group having 1 to 4 carbon atoms, X is an alkylene group having 1 to 4 carbon atoms and optionally being substituted with one or more fluorine atoms, or a carbonyl group -CO-, each of the radicals R³ and R⁴ at the same time are phenyl radicals which may independently be substituted with one or more linear- or branched-chain alkyl or alkoxy radicals having 1 to 4 carbon atoms, or radical R³ is a hydrogen atom or a linear- or branched-chain alkyl radical having 1 to 4 carbon atoms, and radical R⁴ is a 1-naphthyl alkylene radical -(CH₂)ₙ-C₈H₇, a [3-(10,11-dihydro-dibenzo[a,d]cycloheptene-5-ylidene)-propyl] radical -CH₂CH₂CHC₁₅H₁₂, an N-(1-naphthyl)-N-alkylene amine radical -(CH₂)ₙ-NH-C₈H₇, an N-(5H-dibenzo[a,d]cycloheptane-5-yl)-N-alkylene amine radical -(CH₂)ₙ-NH-C₁₅H₁₃, or a radical A as indicated below: 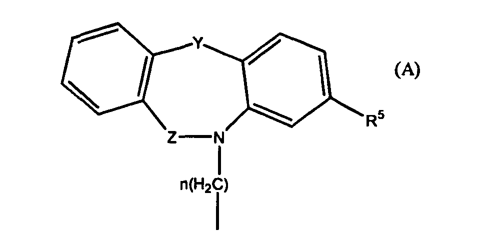 wherein n is 1, 2 or 3, R⁵ is a hydrogen atom or a halogen atom, Z is an alkylene bridge -(CH₂)ₗ-, wherein l equals 0 or 1, Y is an alkylene bridge -(CH₂)ₘ-, wherein m equals 0, 1 or 2, an oxygen atom or an NR⁶ group, wherein radical R⁶ is a hydrogen atom, a linear- or branched-chain alkyl radical having 1 to 4 carbon atoms, or an unsubstituted or substituted aryl radical, or R³ and R⁴ together form a piperazine ring or hexahydropyrimidine ring as indicated below, introducing the nitrogen atom of formula (I): 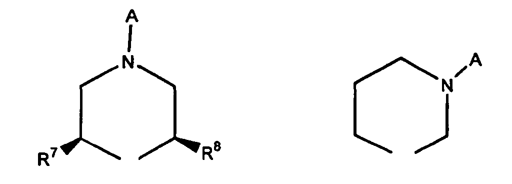 wherein radical A is defined as mentioned above and each of R⁷ and R⁸ independently is a hydrogen atom or an alkyl group having 1 to 4 carbon atoms, or a pharmaceutically acceptable salt thereof for use as a medicament.

2. The compound as claimed in claim 1, whereby radical A is a (10,11-dihydro-dibenzo[b,f]azepine-5-yl)-N-alkylene radical, a (5,6,11,12-tetrahydrodibenzo[b,f]azocine-5-yl)-N-alkylene radical or a carbazole-9-yl-N-alkylene radical.

3. The compound as claimed in claim 1 or 2, whereby radical R³ is a methyl group.

4. The compound as claimed in any of claims 1 to 3, whereby each of the radicals R¹ and R² independently is a hydrogen atom or a methoxy group.

5. The compound as claimed in any of claims 1 to 4, whereby radical R⁵ is a chlorine atom.

6. A pharmaceutical composition comprising a compound as claimed in any of claims 1 to 5 as well as optionally a pharmaceutically acceptable carrier substance and/or a pharmaceutically acceptable diluent.

7. The pharmaceutical composition as claimed in claim 6, whereby the compound having the general formula (I) has a concentration of 4 to 80 weight percent, based on the total amount of the pharmaceutical composition.

8. The pharmaceutical composition as claimed in any of claims 6 or 7, in liquid or solid form or in form of a gel, for administration.

9. The use of a compound having the general formula (II): 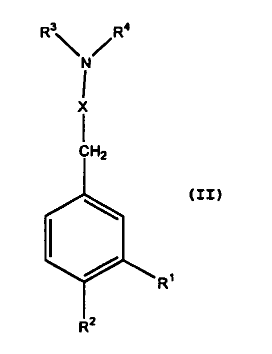 wherein each of the radicals R¹ and R² independently is a hydrogen atom or an alkoxy group having 1 to 4 carbon atoms, X is an alkylene group having 1 to 4 carbon atoms and optionally being substituted with one or more fluorine atoms, or a carbonyl group -CO-, each of the radicals R³ and R⁴ at the same time are phenyl radicals which may independently be substituted with one or more linear- or branched-chain alkyl or alkoxy radicals having 1 to 4 carbon atoms, or radical R³ is a hydrogen atom or a linear- or branched-chain alkyl radical having 1 to 4 carbon atoms, and radical R⁴ is a 1-naphthyl alkylene radical -(CH₂)ₙ-C₈H₇, a [3-(10,11-dihydro-dibenzo[a,d]cycloheptene-5-ylidene)-propyl] radical -CH₂CH₂CHC₁₅H₁₂, an N-(1-naphthyl)-N-alkylene amine radical -(CH₂)ₙ-NH-C₈H₇, an N-(5H-dibenzo[a,d]cycloheptane-5-yl)-N-alkylene amine radical -(CH₂)ₙ-NH-C₁₅H₁₃, or a radical B as indicated below: 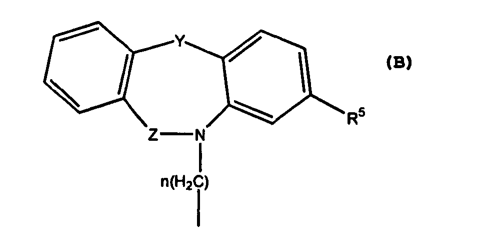 wherein n is 1, 2 or 3, R⁵ is a hydrogen atom or a halogen atom, Z is an alkylene bridge -(CH₂)ₗ-, wherein l equals 0 or 1, Y is an alkylene bridge -(CH₂)ₘ-, wherein m equals 0, 1 or 2, an oxygen atom, a sulfur atom or an NR⁶ group, wherein radical R⁶ is a hydrogen atom, a linear- or branched-chain alkyl radical having 1 to 4 carbon atoms, or an unsubstituted or substituted aryl radical, or R³ and R⁴ together form a piperazine ring or hexahydropyrimidine ring as indicated below, introducing the nitrogen atom of formula (II): 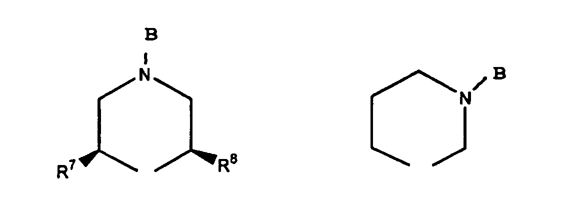 wherein radical B is defined as mentioned above and each of R⁷ and R⁸ independently is a hydrogen atom or an alkyl group having 1 to 4 carbon atoms, or a pharmaceutically acceptable salt thereof for the preparation of a medicament with antiapoptotic and antiseptic effect.

10. The use as claimed in claim 9, for the preparation of a medicament for treating neurodegenerative diseases, sepsis, arteriosclerosis and retroviral infections.

11. The use of a compound as claimed in claim 9 or 10, whereby radical B is a (10,11-dihydro-dibenzo[b,f]azepine-5-yl)-N-alkylene radical, a (5,6,11,12-tetrahydrodibenzo[b,f]azocine-5-yl)-N-alkylene radical, a phenothiazine-10-yl-N-alkylene radical or a carbazole-9-yl-N-alkylene radical.

12. The use of a compound as claimed in claims 9 to 11, whereby radical R³ is a methyl group.

13. The use of a compound as claimed in any of claims 9 to 12, whereby each of the radicals R¹ and R² independently is a hydrogen atom or a methoxy group.

14. The use of a compound as claimed in any of claims 9 to 13, whereby radical R⁵ is a chlorine atom.

## Revendications

1. Composé de la formule générale (I) 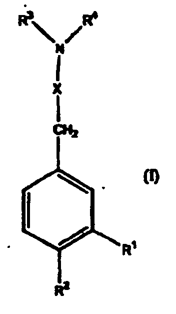 dans laquelle les radicaux R¹ et R² représentent à chaque fois, indépendamment l'un de l'autre, un atome d'hydrogène ou un radical alcoxy comportant de 1 à 4 atomes de carbone, X représente un radical alkylène comportant de 1 à 4 atomes de carbone, qui peut être substitué par un ou plusieurs atomes de fluor, ou un groupe cétone -CO-, les radicaux R³ et R⁴ représentent à chaque fois en même temps des radicaux phényle qui peuvent, indépendamment l'un de l'autre, être substitués par un ou plusieurs radicaux alkyle ou alcoxy à chaîne linéaire ou ramifiée, comportant de 1 à 4 atomes de carbone, ou bien le radical R³ représente un atome d'hydrogène ou un radical alkyle à chaîne linéaire ou ramifiée et comportant de 1 à 4 atomes de carbone, et le radical R⁴ un radical 1-naphtylalkylène -(CH₂)ₙ-C₈H₇, un radical [3-(10,11-dihydro-dibenzo[a,d]cycloheptén-5-ylidène)-propyle] CH₂CH₂CHC₁₅H₁₂, un radical N-(1-naphtyl)-N-alkylène-amine (CH₂)ₙ-NH-C₈H₇, un radical N-(5H-dibenzo[a,d]cycloheptan-5-yl)-N-alkylène-amine (CH₂)ₙ-NH-C₁₅H₁₃, ou un radical A ci-après, 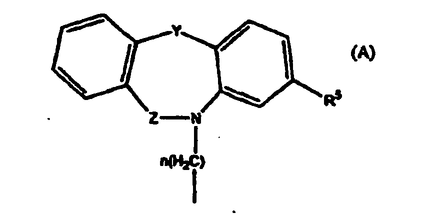 dans lequel n vaut 1, 2 ou 3, R⁵ représente un atome d'hydrogène ou un atome d'halogène, Z un pont alkylène -(CH₂)ₗ où l = 0 ou 1, Y un pont alkylène -(CH₂)ₘ, où m = 0, 1 ou 2, un atome d'oxygène ou un groupe NR⁶, où le radical R⁶ représente un atome d'hydrogène, un radical alkyle à chaîne linéaire ou ramifiée comportant de 1 à 4 atomes de carbone ou un radical aryle substitué ou non substitué, ou bien R³ et R⁴ forment ensemble, avec combinaison de l'atome d'azote de la formule (I), un noyau de pipérazine ou un noyau d'hexahydropyrimidine ci-après 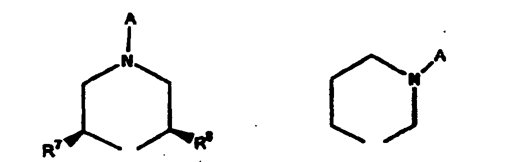 où le radical A est défini comme ci-avant et R⁷ et R⁸ représentent à chaque fois, indépendamment l'un de l'autre, un atome d'hydrogène ou un radical alkyle comportant de 1 à 4 atomes de carbone, ou un sel pharmaceutiquement compatible de celui-ci, aux fins d'utilisation en tant que médicament.

2. Composé selon la revendication 1, dans lequel le radical A est un radical (10,11-dihydro-dibenzo[b,f]azépin-5-yl)-N-alkylène, un radical (5,6,11,12-tétrahydro-dibenzo[b,f]azocin-5-yl)-N-alkylène ou un radical carbazol-9-yl-N-alkylène.

3. Composé selon la revendication 1 ou 2, dans lequel le reste R³ est un radical méthyle.

4. Composé selon l'une quelconque des revendications 1 à 3, dans lequel les radicaux R¹ et R² représentent à chaque fois, indépendamment l'un de l'autre, un atome d'hydrogène ou un radical méthoxy.

5. Composé selon l'une quelconque des revendications 1 à 4, dans lequel le radical R⁵ est un atome de chlore.

6. Composition pharmaceutique contenant un composé selon l'une quelconque des revendications 1 à 5 et le cas échéant un excipient pharmaceutiquement compatible et/ou un agent de dilution pharmaceutiquement compatible.

7. Composition pharmaceutique selon la revendication 6, dans lequel le composé de la formule générale (I) est présent en une concentration de 4 à 80% en poids, par rapport à la quantité totale de la composition pharmaceutique.

8. Composition pharmaceutique selon l'une quelconque des revendications 6 ou 7, se présentant sous une forme d'administration liquide, gélatineuse ou solide.

9. Utilisation d'un composé de la formule générale (II) 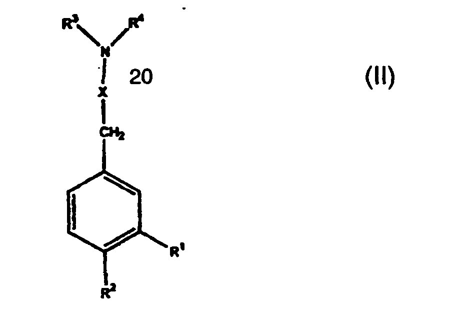 dans laquelle les radicaux R¹ et R² représentent à chaque fois, indépendamment l'un de l'autre, un atome d'hydrogène ou un radical alcoxy comportant de 1 à 4 atomes de carbone, X représente un radical alkylène comportant de 1 à 4 atomes de carbone, qui peut être substitué par un ou plusieurs atomes de fluor, ou un groupe cétone -CO-, les radicaux R³ et R⁴ représentent à chaque fois en même temps des radicaux phényle qui peuvent, indépendamment l'un de l'autre, être substitués par un ou plusieurs radicaux alkyle ou alcoxy à chaîne linéaire ou ramifiée, comportant de 1 à 4 atomes de carbone, ou bien le radical R³ représente un atome d'hydrogène ou un radical alkyle à chaîne linéaire ou ramifiée et comportant de 1 à 4 atomes de carbone, et le radical R⁴ un radical 1-naphtylalkylène -(CH₂)ₙ-C₈H₇, un radical [3-(10,11-dihydro-dibenzo[a,d]cycloheptén-5-ylidène)-propyle] CH₂CH₂CHC₁₅H₁₂, un radical N-(1-naphtyl)-N-alkylène-amine (CH₂)ₙ-NH-C₈H₇, un radical N-(5H-dibenzo[a,d]cycloheptan-5-yl)-N-alkylène-amine (CH₂)ₙ-NH-C₁₅H₁₃, ou un radical B ci-après, 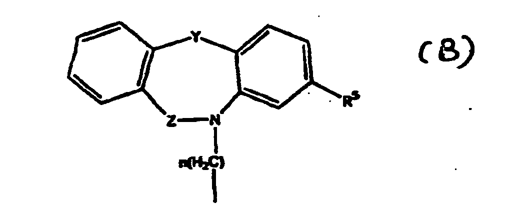 dans lequel n vaut 1, 2 ou 3, R⁵ représente un atome d'hydrogène ou un atome d'halogène, Z un pont alkylène -(CH₂)_{L} où l = 0 ou 1, Y un pont alkylène -(CH₂)ₘ, où m = 0, 1 ou 2, un atome d'oxygène, un atome de soufre ou un groupe NR⁶, où le radical R⁶ représente un atome d'hydrogène, un radical alkyle à chaîne linéaire ou ramifiée comportant de 1 à 4 atomes de carbone ou un radical aryle substitué ou non substitué, ou bien R³ et R⁴ forment ensemble, avec combinaison de l'atome d'azote de la formule (II), un noyau de pipérazine ou un noyau d'hexahydropyrimidine ci-après 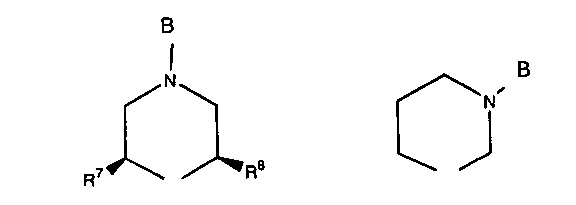 où le radical B est défini comme ci-avant et R⁷ et R⁸ représentent à chaque fois, indépendamment l'un de l'autre, un atome d'hydrogène ou un radical alkyle comportant de 1 à 4 atomes de carbone, ou un sel pharmaceutiquement compatible de celui-ci, pour la préparation d'un médicament à activité antiapoptotique et antiseptique.

10. Utilisation selon la revendication 9 pour la préparation d'un médicament destiné au traitement de maladies neurodégénératives, de sepsies, de l'artériosclérose et d'infection à rétrovirus.

11. Utilisation d'un composé selon la revendication 9 ou 10, où le radical B est un radical (10,11)dihydro-dibenzo[b,f]azépin-5-yl)-N-alkylène, un radical (5,6,11,12-tétrahydrodibenzo[b;f]azocinyl-5-yl)-N-alkylène, un radical phénothiazin-10-yl-N-alkylène ou un radical carbazol-9-yl-N-alkylène.

12. Utilisation d'un composé selon les revendications 9 à 11, où le radical R³ est un radical méthyle.

13. Utilisation d'un composé selon l'une quelconque des revendications 9 à 12, où les radicaux R¹ et R² sont à chaque fois, indépendamment l'un de l'autre, un atome d'hydrogène ou un radical méthoxy.

14. Utilisation d'un composé selon l'une quelconque des revendications 9 à 13, où le radical R⁵ est un atome de chlore.
